# EUROPEAN PATENT SPECIFICATION

(11) **EP 3 270 906 B1**
(45) Date of publication and mention of the grant of the patent: **05.06.2019**
(21) Application number: 16708701.4
(22) Date of filing: 09.03.2016
(51) Int. Cl.: A61K 31/164, A61K 31/197, A61K 31/455, A61K 31/51, A61K 31/525, A61K 31/714, A61P 25/00

(54) **COMPOSITION FOR USE IN THE TREATMENT OF NEUROPATHIC PAIN**
ZUSAMMENSETZUNG ZUR VERWENDUNG BEI DER BEHANDLUNG VON NEUROPATHISCHEM SCHMERZ
COMPOSITION SERVANT AU TRAITEMENT DE LA DOULEUR NEUROPATHIQUE

(30) Priority: 16.03.2015 NL 2014464
(43) Date of publication of application: 24.01.2018
(73) Proprietor: Vanderbilt Science Holding Ltd., 1075 Nicosia (CY)
(72) Inventor: KRIEK, Rutger, 1075 Nicosia (CY)
(74) Representative: Nederlandsch Octrooibureau
(86) International application number: PCT/EP2016/055035
(87) International publication number: WO 2016/146453

(56) References cited:
- NL-A- 2 011 448
- US-A1- 2011 171 313
- KEPPEL J M H ET AL: "Therapeutic utility of palmitoylethanolamide in the treatment of neuropathic pain associated with various pathological conditions: A case series", JOURNAL OF PAIN RESEARCH 2012 DOVE MEDICAL PRESS LTD. NZL, vol. 5, 2012, pages 437-442, XP002757426, ISSN: 1178-7090
- STRACKE H ET AL: "A BENFOTIAMINE-VITAMIN B COMBINATION IN TREATMENT OF DIABETIC POLYNEUROPATHY", EXPERIMENTAL AND CLINICAL ENDOCRINOLOGY AND DIABETES, JOHANN AMBROSIUS BARTH VERLAG IN MEDIZINVERLAGE HEIDELBERG GMBH & CO. KG, DE, vol. 104, no. 4, 1 January 1996 (1996-01-01), pages 311-316, XP009036456, ISSN: 0947-7349
- BEHRENDS ET AL: "Vitamin-B-Komplex lindert Nervenschmerz. Diabetes - periphere Polyneuropathie [Vitamin B complex for the alleviation of pain in diabetic polyneuropathy]", FORTSCHRITTE DER MEDIZIN, URBAN UND VOGEL, MUENCHEN, DE, vol. 109, no. 11, 1 January 1991 (1991-01-01), pages 63-64, XP008180029, ISSN: 0015-8178
- PETERS T J ET AL: "Treatment of alcoholic polyneuropathy with vitamin B complex: A randomised controlled trial", ALCOHOL AND ALCOHOLISM, vol. 41, no. 6, November 2006 (2006-11), pages 636-642, XP002757427, ISSN: 0735-0414

## Description

### TECHNICAL FIELD OF THE INVENTION

The present invention relates to a composition comprising palmitoylethanolamide (PEA) and a vitamin B. The present invention also relates to such a composition for use as a nutraceutical or use as a medicament, for use as an analgesic pharmaceutical, and more specifically for use as an analgesic pharmaceutical in the alleviation of neuropathic pain, and to a method for preparing such a composition.

### BACKGROUND OF THE INVENTION

Various diseases or conditions, including viral infections, such as HIV infections and herpes zoster infections, mammectomy, amputation, chemotherapy, axonal neuropathies, diabetes and complex regional pain syndrome, cause difficult to treat pain. For many cases, these pains are classified as neuropathic pain. For example, post-herpetic neuralgia is caused by a herpes zoster infection and causes moderate to severe neuropathic pain in the infected skin area of the subject. Problems encountered in management of such neuropathic pain is a far from optimal balance between effectiveness obtainable by the use of current therapeutic compositions and accompanying issues related to side effects and safety issues.

Indeed, currently, various drugs are used to combat neuropathic pain, though without reaching the desired level of success for the patient. Side effects induced by current prescribed pain-relieving drugs limit their use by making it impossible to reach effective dose levels. So, there is a need for effective and safe treatment options for patients suffering from neuropathic pain. Accordingly, research continues into new or alternative methods for treating neuropathic pain in a manner that is long lasting, effective, with few side effects and good tolerability.

Anti-depressants reduce some types of neuropathic pain, to some extent. Their partial efficacy has been demonstrated for neuropathic pain caused by diabetes and trauma, post herpetic neuralgia, and spinal cord damage. However, taking anti-depressants is associated with significant dose-limiting side effects, including cardiac disturbances, urinary retention, drowsiness, dizziness and orthostatic hypotension.

Gabapentinoids have a general depressant action on neuronal hyper-excitability, a mechanism that is common to many chronic pain conditions including neuropathic pain. Gabapentin and pregabalin are two widely used members of this class of compounds. Unfortunately, many patients have to stop their pain treatment with a gabapentinoid due to unacceptable side effects such as drowsiness, dizziness and peripheral edema.

Opioids are effective to some extent in reducing the intensity of some neuropathic pain states. However, these drugs commonly cause side effects expressed as cognitive ability, constipation and nausea, and their use is further limited by the risk of abuse of the opioid.

In summary, several members of the commonly prescribed drugs of the classes of anti-depressants, gabapentinoids and opioids are only partial effective at best, when treatment of pain is considered, achieving only a partial reduction of pain: 25- 40% pain reduction in 40-60% of patients. Moreover, application of these drugs in neuropathic pain treatment regimens is accompanied by numerous undesirable side effects, which limit, or even prohibit, their use.

Due to increasing insight in the complex patho-mechanisms underlying chronic and neuropathic pain, more and more combinations of analgesic compounds are prescribed to neuropathic pain patients. However, such combinations are based on combining two or more pharmaceutical compounds of the classes of anti-epileptics (for example pregabalin, gabapentin), anti-depressants (*e.g*. amitriptyline, duloxetine), non-steroidal analgesic anti-inflammatory compounds (*e.g.* ibuprofen), and opioids (*e.g.* tramadol, oxycodin), all with their far from optimal safety profile. Due to the side effects, related for example to the inhibition of neuronal activity, most patients indeed heavily suffer when treated with such combinations (drowsiness, confusion, impairment of driving skills).

In a search for improved efficacy accompanied with less side effects and a better safety profile, attention was focused on therapy combining the aforementioned analgesics with a stimulatory co-factor compound having an acceptable safety profile and being able to enhance the pain relieving activity of the analgesic, potentially allowing for the application of a lower effective dose of the analgesic.

Compositions comprising anti-depressant agomelatine or a different member of agomelatine related compounds together with palmitoylethanolamide (PEA) as a co-factor, have been described in international patent application WO2013063263. In this application, a pharmaceutical composition for the prevention or treatment of neuropathic pain is disclosed, wherein the composition comprises an agomelatine-like naphthalene compound, such as agomelatine itself, together with PEA.

Patients suffering from neuropathic pain and previously treated with analgesic only, have subsequently been treated with the analgesics combined with PEA as a co-factor (J. Keppel Hesselink & T. Hekker, J. Pain Res. 2012:5 437-442).

An analgesic effect is seen in patients on gabapentin therapy now in combination with vitamins from the B-complex family as a co-factor for enhancing gabapentin efficacy (R. Medina-Santillan et al., Proc. West. Pharmacol. Soc. 47: 109-112 (2004)). Treatment of patients with gabapentin is associated with a high frequency of adverse effects.

Thus, although some improvements related to enhanced efficacy were observed, treatment of patients with analgesic drug compounds known for their high risk for side effects and unfavorable safety profile, is still continued.

Concluding, all of these aforementioned compositions comprising prescribed drugs with analgesic activity do not provide for a highly effective relieve of pain in chronic pain patients, including patients suffering from neuropathic pain, accompanied with good tolerability at an effective dose and a good safety profile. Thus, there exists a need for compositions which are effective in sufficiently relieving neuropathic pain, accompanied by an acceptable safety profile and with no or minimal side effects, which does not hamper reaching the aimed effective dose.

### SUMMARY OF THE INVENTION

A first aspect of the present invention relates to a composition comprising palmitoylethanolamide and one or more vitamins of the vitamin B complex or salts thereof, wherein the composition comprises between 30% and 3000% of the recommended daily intake of vitamin B, preferably between 50% and 1000% per unit of composition.

Palmitoylethanolamide (PEA) is an endogenous fatty acid amide. PEA is classified as a food for medical purposes, and as a dietary supplement. Vitamin B complex is a class of endogenous vitamins, also present in various food products.

Therefore, a preferred embodiment of the present invention relates to a composition comprising PEA and one or more vitamins of the vitamin B complex or salts thereof, wherein the composition is a pharmaceutical composition or a nutraceutical composition.

Preferably, PEA and the one or more vitamins of the vitamin B complex or salts thereof are the only pharmaceutically active ingredients in the composition according to the invention.

Both PEA and the vitamins of the vitamin B complex are in separate occasions applied as a co-factor in pharmaceutical compositions comprising an analgesic compound. The current invention now provides for pharmaceutical compositions containing or comprising both co-factors PEA and one or more vitamins of the vitamin B complex or salts thereof, together.

A second aspect of the present invention relates to a method for preparing the composition according to the present invention.

A third aspect of the present invention relates to a composition comprising PEA and one or more vitamins of the vitamin B complex or salts thereof, for use as a medicament.

Compositions of the invention comprising PEA and one or more vitamins of the vitamin B complex or salts thereof, are particularly suitable for use in the alleviation of pain, especially the alleviation of neuropathic pain.

Therefore, a further aspect of the invention relates to a composition comprising PEA and one or more vitamins of the vitamin B complex or salts thereof, for use in pain alleviation, preferably neuropathic pain alleviation.

### DEFINITIONS

The term "neuropathic pain" as used herein has its conventional meaning and has been defined by the International Association for the Study of Pain (IASP, 2011) as 'Pain caused by a lesion or disease of the somatosensory nervous system'. The IASP further specifies: 'Neuropathic pain is a clinical description (and not a diagnosis) which requires a demonstrable lesion or a disease that satisfies established neurological diagnostic criteria. The presence of symptoms or signs (*e.g*., touch-evoked pain) alone does not justify the use of the term neuropathic. Some disease entities, such as trigeminal neuralgia, are currently defined by their clinical presentation rather than by diagnostic testing. Other diagnoses such as post-herpetic neuralgia are normally based upon the history.

Neuropathic pain can be divided according to the IASP in two different pain states:
1. Central neuropathic pain, defined by the IASP as 'pain caused by a lesion or disease of the central somatosensory nervous system'; and
2. Peripheral neuropathic pain, defined by the IASP as 'pain caused by a lesion or disease of the peripheral somatosensory nervous system'.

The term "lesion" as used herein has its conventional meaning and refers to a situation when diagnostic investigations (*e.g.* imaging, neurophysiology, biopsies, lab tests) reveal an abnormality or when there was obvious trauma.

The term "vitamin" as used herein has its conventional meaning and includes salts of such vitamins.

The term "somatosensory" as used herein has its conventional meaning and refers to information about the body per se including visceral organs, rather than information about the external world related to the body (*e.g.,* vision, hearing, or olfaction).

The term "synergy" as used herein has its conventional meaning and refers to two or more compounds that interact in a way that enhance or magnify one or more effects of those compounds.

The term "optimal" in the context of "optimal tolerability" as used herein has its conventional meaning and refers to an amount of a compound administered to a subject, which effectively reliefs pain in the subject to a desired level, whereas side-effects stay below an acceptable threshold.

The term "dose-limiting side-effects" as used herein has its conventional meaning and refers to unacceptable side effects that would occur in a subject when a dose of a compound would be administered, high enough for effective pain relief in a subject to a desired level.

The term "classical analgesic drug" as used herein has its conventional meaning and refers to the types of analgesic drugs commonly prescribed to neuropathic pain patients up to the date of filing of the current application and up to the priority date.

The term "recommended daily allowances" ("RDA"), "Recommended daily intake" ("RDI") or "Recommended daily dose" ("RDD"), as used herein have their conventional meaning in the context of daily vitamins uptake by a human. The values listed in the current specification are according to standards of the European Union (Commission Directive 2008/100/EC). These RDA are 1,1 mg for vitamin B1, 1,4 mg for vitamin B2, 16 mg for vitamin B3, 6 mg for vitamin B5, 1,4 mg for vitamin B6, 50 µg for vitamin B8, 200 µg for folic acid and 2,5 µg for vitamin B12.

The term "vitamin B complex" has its conventional meaning and refers to the water soluble vitamins B1 (Thiamine), B2 (Riboflavin), B3 (Niacin), B5 (Pantothenic acid), B6 (pyridoxine, pyridoxal, pyridoxamine), B7 (Biotin), B9 (Folic acid) and B12 (Cobalamin).

The term "recommended daily intake of vitamin B", has its conventional meaning and refers to the amount of the one or more family members of the vitamin B complex present in a dose or a daily dose of a composition according to the invention.

The term "dosage" as used herein has its conventional meaning and refers to the giving of a single dose of a compound to a subject.

The term "daily dosage" as used herein has its conventional meaning and refers to the giving of a total dose of a compound to a subject per day, administered as a single dose, or as several doses throughout the day.

The term "unit of composition" as used herein shall mean a ready to use unit of composition in any form, either as food product, a drink, a food additive, a food supplement, or as a formulation, such as a tablet, a chewing tablet, a capsule or a liquid, a suspension, or a sublingual formulation or any formulation for mucosal absorbance of the active ingredients of said composition, such as a lozenge or a quick dissolving powder, or a transdermal or topical formulation wherein the composition is administered in a cream, an ointment or a gel.

The term a "single compounded cream or gel" as used herein has its conventional meaning and refers to a cream or a gel comprising two or more active ingredients mixed together in a cream or gel, thereby providing a multi-compound compounded pharmaceutical or nutraceutical.

The term "fixed-dose combination" as used herein has its conventional meaning and refers to the bulk fixed-dose combination as well as single dosages of the fixed-dose combination, retrieved from this bulk.

The terms "pharmaceutically acceptable" and "nutraceutically acceptable" as used herein have their conventional meaning and refer to compounds, material, compositions and/or dosage forms, which are, within the scope of sound medical judgment suitable for contact with the tissues of mammals, especially humans, without excessive toxicity, irritation, allergic response and other problem complications commensurate with a reasonable benefit/risk ratio.

The term "salt" as used herein has its conventional meaning and includes the acid addition and base salts.

The term "treatment" as used herein has its conventional meaning and refers to curative, palliative and prophylactic treatment.

The term "only active ingredient" as used herein has its conventional meaning and means that a given compound is present as the sole pharmaceutically active ingredient and that no other compounds are present in an amount which render a pharmaceutically relevant effect in the human or animal body.

### BRIEF DESCRIPTION OF THE DRAWING

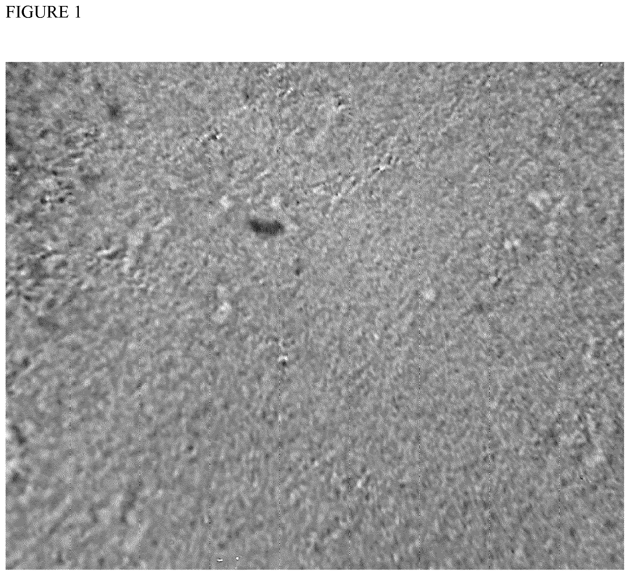

FIGURE 1: microscopic photograph of a fat in (olive)-oil matrix of palmitoylethanolamide (10%: palmitoylethanolamide dissolves in hot oil resulting in a semi-solid fatty-oil matrix).

### DETAILED DESCRIPTION OF THE PRESENT INVENTION

Numerous attempts have been conducted to overcome the current drawbacks seen with classical analgesic drugs when applied in the treatment of pain, *e.g.* neuropathic pain, though with limited success. Neuropathic pain patients treated with a classical analgesic drug or with an analgesic drug from the classes of anti-depressants, gabapentinoids and opioids, commonly suffer from adverse effects, while efficacy of the treatment is in many occasions suboptimal at best.

Following one line of reasoning, in order to reach an effective dose of an analgesic drug, however accompanied with acceptable side effects, co-stimulatory factors were searched for. These co-stimulatory factors, or "co-factors", ideally enhance the efficacy of the analgesic drug in such a way that relief of pain is reached in the patient already at a drug dose at which dose-limiting side-effects do not yet hamper the treatment.

Co-factors that were analyzed for their co-stimulatory activity towards analgesic drugs are, amongst others, palmitoylethanolamide (PEA) and, in separate research programs, vitamins belonging to the vitamin B group (or vitamin B-complex group). Although providing some improvement, an efficacious drug for the treatment of neuropathic pain, without side effects, has not been obtained until now.

In order to overcome the sub-optimal ratio between treatment efficacy and adverse effects due to the treatment, a novel composition omitting classical analgesic drugs is developed by the current inventors. It is surprisingly found that combining PEA and a vitamin belonging to the vitamin B group (or vitamin-B complex group) as the only pharmaceutically active ingredients, previously separately known for their co-factor activity towards analgesic drugs, provided a composition according to the invention with improved analgesic efficacy already only by themselves, and thus when omitting the incorporation of a classical analgesic drug in the composition of the invention. Moreover, the inventors found that treatment of patients with such a composition comprising the two co-factors PEA and a vitamin B did not induce the side-effects commonly seen when patients are treated with a classical analgesic drug. In addition, surprisingly, such a composition comprising the two co-factors PEA and a vitamin B results in a much faster onset of action, when administered to a patient.

Therefore, a first aspect of the present invention relates to a composition comprising PEA and one or more vitamins of the vitamin B complex or salts thereof. According to the present invention, both PEA and one or more vitamins of the vitamin B complex or salts thereof are now used as the pharmaceutically active ingredients and no longer as co-factors.

In one embodiment of the present invention, PEA and the one or more vitamins of the vitamin B complex or salts thereof are the only pharmaceutically active ingredients present in the composition.

PEA belongs to a group of endogenous molecules (lipid autacoids) known as N-acylethanolamines. PEA has a beneficial pharmacological profile based on its anti-inflammatory, analgesic and neuroprotective properties. PEA is described in patent application EP0550006 as a compound able to down regulate overactive mast cells, and international patent application WO2001010434 describes micronized pharmaceutical compositions containing PEA. A pharmaceutical formulation containing PEA, co-ultramicronized with antioxidants such as quercetin, resveratrol, polydatin, luteolin, tocopherol, and thioctic acid, is known based on patent application US20110171313: 'Composition containing ultra-micronized palmitoyl-ethanolamide.' The addition of such antioxidants, however, has never been clinically proven to bring synergies for efficacy, and most of these antioxidants, especially the antioxidants of herbal origin, do not have well known safety profiles.

The family of B vitamins is a class of water-soluble vitamins that play important roles in cell metabolism. B vitamin family members include vitamin B1 (thiamine), vitamin B2 (riboflavin), vitamin B3 (niacin or niacin-amide), vitamin B5 (pantothenic acid), vitamin B6 (pyridoxine, pyridoxal, pyridoxamine), vitamin B7 (biotin), vitamin B9 (folic acid) and vitamin B12 (various cobalamins; such as cyanocobalamin).

Preferably, the composition according to the present invention comprises PEA and one or more vitamins of the vitamin B complex or a salts thereof, wherein the one or more vitamins of the vitamin B complex is, or respectively, are chosen from vitamin B1, vitamin B2, vitamin B3, vitamin B5, vitamin B8, folic acid, vitamin B12, or a combination thereof. Typically, the compositions of the invention comprise one or more vitamins of the vitamin B complex at a dose expressed as a percentage of the recommended daily allowances per unit. The compositions of the invention comprise between 30% and 3000% of the recommended daily intake of vitamin B, and more preferably between 50% and 1000%, most preferably between 20% and 500%, per unit of composition.

In patients suffering from chronic idiopathic axonal neuropathy, dosages of 400 mg PEA, administered thrice per day, 600 mg PEA administered twice per day, and 2400 mg PEA, administered once a day, were shown to be effective and well tolerated by the patients. Therefore, suitable compositions comprising an effective dose of PEA according to the invention, comprise between 100 and 800 mg PEA, preferably between 300 mg and 600 mg PEA per unit of composition. Thus, an important aspect of the present invention is a composition comprising PEA and one or more vitamins of the vitamin B complex or salts thereof, wherein the composition is a pharmaceutical composition or a nutraceutical composition. An equally important aspect of the invention is a composition comprising PEA and one or more vitamins of the vitamin B complex or a salts thereof, wherein the composition is a food product, a drink, a food additive, a food supplement or a food ingredient.

It is now due to the current invention that it has been found that a composition comprising both PEA and one or more vitamins of the vitamin B complex or salts thereof, is an efficacious combination drug, even when omitting a classical drug. Thus, for the first time, a combination drug of PEA and at least one vitamin B, or a salt thereof as the only pharmaceutically active ingredients, has now become available as a medicament.

In one embodiment the composition according to the present invention comprises between 100 and 800 mg PEA, preferably between 300 mg and 600 mg PEA, per unit of composition; optionally, containing said PEA in an oil matrix, in a volume ratio of 5%-20% (volume/volume), preferably 10% (volume/volume) PEA / olive oil.

The composition according to the present invention may also comprise an oil such as a vegetable oil or a fish oil and/or the composition according to the present invention may also comprise vitamin D. Preferably, the vegetable oil or the fish oil contains omega-3 fatty acids. Preferably, such a composition comprises between 50 and 150 mg of a vegetable oil or a fish oil, per unit of composition. The vitamin D preferably ranges between 10 and 40 microgram vitamin D3, per unit of composition.

The composition according to the present invention has preferably been formulated as a pharmaceutical composition or as a nutraceutical composition.

Furthermore, the composition according to the present invention has preferably been formulated as an oral formulation, such as a tablet, chewing tablet, a capsule, a liquid or a suspension. The composition according to the present invention may also be formulated as a sublingual formulation or any formulation suitable for mucosal absorbance of the active ingredients of said composition, such as a lozenge or a quick dissolving powder. Alternatively, the composition according to the present invention may be formulated as a transdermal or topical formulation, wherein the composition is administered as a cream, an ointment or a gel.

Preferably, the composition has been formulated as a single compounded cream or gel comprising a cream basis or gel basis selected from Cremor cetomacrogolis FNA or Lanette cream FNA.

In an alternative embodiment, the composition according to the present invention comprises one or more analgesic compounds, besides PEA and the one or more vitamins of the vitamin B complex or salts thereof. Preferably, such composition comprises 1-20% by weight analgesic compounds.

Preferred analgesic compounds are ketamine ((RS)-2-(2-Chlorophenyl)-2-(methylamino)cyclohexanone), gabapentin (1-(aminomethyl)cyclohexaneacetic acid), pregabalin ((S)-3-(aminomethyl)-5-methylhexanoic acid), amitriptyline (3-(10,11-Dihydro-5H-dibenzo[a,d]cycloheptene-5-ylidene)-N,N-dimethylpropan-1-amine), duloxetine ((+)-(S)-N-Methyl-3-(naphthalen-1-yloxy)-3-(thiophen-2-yl)propan-1-amine), baclofen ((RS)-4-Amino-3-(4-chlorophenyl)butanoic acid) or a combination of any of these analgesic compounds.

A second aspect of the present invention relates to a method for preparing the compositions as has been described above.

Preferably, the PEA and one or more vitamins of the vitamin B complex or salts thereof have been granulated into particles with an average particle size (D50) of between 10 and 1000 micron (micrometer), preferably between 10 and 100 micron (micrometer). In the art, several methods are readily available to the skilled person to prepare particles with the above mentioned particle size.

The method according to the present invention, preferably comprises the step of adding the PEA particles to an oil, preferably any vegetable oil or any fish oil comprising omega-3 fatty acids, preferably olive oil, such that a composition comprising PEA in an oil matrix, in a volume ratio of 5%-20% (volume/volume), preferably 10% (volume/volume) PEA / vegetable oil or PEA / fish oil, containing omega-3 fatty acids, preferably PEA / olive oil, is obtained, wherein the oil is with or without enrichment with omega-3-fatty acids.

A further aspect of the invention relates to a composition comprising PEA and one or more vitamins of the vitamin B complex or salts thereof, for use as a medicament, preferably for use in pain alleviation.

In this regard, it is noted that preferably, no other pharmaceutically active compounds are present in the composition.

It is now due to the achievements of the present inventors that an efficacious and safe treatment of patients suffering from pain, without undesired side effects, has become available. Thus, the composition according to the invention is efficiently used in the treatment of pain.

Hence, the composition according to the present invention is preferably used in pain alleviation.

Since in particular pain alleviation in neuropathic patients is hampered by limited treatment options with reasonable efficacy and acceptable side effects, the current invention particularly provides a composition for use in neuropathic pain alleviation.

One of the advantages of a composition according to the invention is effective treatment of pain, especially neuropathic pain, without side-effects commonly hampering the use of classical analgesic drugs. One other advantage of a composition according to this invention over a fixed combination between PEA and co-factors such as antioxidants, is the long-standing safety profile of one or more vitamins of the vitamin B complex or salts thereof.

Since neuropathic pain is often the result of an underlying disease or health problem, the compositions according to the invention are suitable for relieving the neuropathic pain in patients suffering from such diseases or health problems. Examples of diseases and health problems accompanied by neuropathic pain that is effectively treated by the compositions according to the invention, are an axonal neuropathy such as diabetic neuropathy, a demyelinating neuropathy, chemotherapy induced neuropathy, HIV neuropathy, post herpetic neuropathy or postoperative pain.

The compositions according to the invention exhibit synergies between PEA and one or more vitamins belonging to the B vitamin family, providing analgesic efficacy in the absence of troublesome CNS related side effects or other tolerability issues. Various embodiments of the invention disclose a number of effective, tolerable and safe combinations of PEA and compounds belonging to the B vitamin family, based on the endogenous molecule PEA and one or more vitamins from the B complex family.

Topical creams consisting of pyridoxamine (vitamin B6) are well known in the art. These creams, however, are not for treatment of neuropathic pain.

Without wishing to be bound by any theory, the mechanisms underlying the inhibition of neuropathic pain by the use of B vitamins according to the invention are thought to be inhibition of the diacylglycerol protein kinase C (PKC) pathway, enhanced cGMP production by guanylylcyclase enzyme and increased afferent inhibitory control of nociceptive neurons at the spinal cord (Fu GQ, Carstens E, Stelzer B, Zimmermann M. B vitamins suppress spinal dorsal horn nociceptive neurons in the cat. Neurosci. Lett. 1998;95:3192-7), all of which can act complementary and synergistically to the mechanisms of action of PEA. In turn, PEA mainly acts via the peroxisome proliferator-activated receptor (PPAR)-alpha receptor, and via modulation of non-neuronal cells such as the glia- and the mast cells, as well as via a number of additional non-neuronal related mechanisms.

Niacin belongs to the class of B vitamins and is known for its general analgesic properties (Kamerath JH, De Luigi AJ. Analgesic benefit of niacin for shrapnel wound pain in war veteran. Mil. Med. 2010 Nov;175(11):876-7.). Niacin has been used as adjunctive therapy for the treatment of pain conditions such as migraine headaches (Veiling DA, Dodick DW, Muir JJ. Sustained-release niacin for prevention of migraine headache. Mayo Clin. Proc. 2003 Jun;78(6):770-1.). Niacin also has been described to reduce neuropathic pain.

There are a number of natural compounds, such as unsaturated oils and vitamin D which can add to the synergies described in this invention. Vitamin D and long-chain polyunsaturated fatty acids (PUFA) both are involved in controlling inflammatory and immune responses. Borage oil for instance is known to be rich in the gamma-linolenic acid (26%-38%) which in itself is used as dietary or food supplement. (Asadi-Samani M. et al., Asian Pac. J. Trop. Med. 2014 Sep;7S1:S22-8.) Dietary supplementation with borage oil, flaxseed oil, and echium oil affects the biochemistry of fatty acid metabolism and are considered to hold great promise for modulating inflammatory diseases. (Chilton FH. et al., Am. J. Clin. Nutr. 2008 Feb;87(2):498S-503S). In addition it has become clear that a number of the biochemical properties of Extra-Virgin Olive Oil are linked to its anti-inflammatory properties. (Takashima T. et al., The Journal of Nutritional Biochemistry 2014; 25: 186-192) Vitamin D has been described recently to also encompass biological relevant anti-inflammatory properties. (Li YC. et al., J. Steroid Biochem. Mol. Biol. 2015 Jan 17.) In an alternative embodiment of the composition of the present invention, the composition therefore further comprises a vegetable oil, such as borage oil, olive oil or echium seed oil or a non-vegetable oil such as fish oil with or without vitamin D.

In an alternative embodiment of the composition of the present invention, the composition further comprises an analgesic drug selected from the classes of antidepressants, anti-epileptics or opioids. In a specific embodiment of the present invention the anti-epileptic compound is selected from pregabalin and gabapentin. Preferably, the analgesic drug is selected from duloxetine ((+)-(S)-N-methyl-3-(1-naphtyloxy)-3-(2-thienyl)propylamine), pregabalin ((S)-3-aminomethyl-5-methyl-caproic acid), tramadol (2-[(Dimethylamino)methyl]-1-(3-methoxyphenyl)cyclohexanol) and tapentadol (3-[(1R,2R)-3-(dimethylamino)-1-ethyl-2-methylpropyl]phenol hydrochloride). Preferably, the analgesic drug is selected from amitriptyline (3-(10,11-Dihydro-5H-dibenzo[a,d]cycloheptene-5-ylidene)-N,N-dimethylpropan-1-amine), gabapentin and oxycontin (5R,9R,13S,14S)-4,5α-epoxy-14-hydroxy-3-methoxy-17-methylmorphinan-6-one).

If the treatment regimens according to current practice, consisting of administering above listed classical analgesics to the neuropathic pain patient, was expanded with picamilon, and with PEA, enhanced analgesic properties arose, as will be demonstrated in some of the examples below.

Furthermore, use of compositions according to the invention for the treatment of patients suffering from neuropathic pain results in improvements: patients' mood was lifted, anxiety decreased, sleep and quality of life was improved. An example of such an effective composition according to the invention is described in example 1. When applied separately, PEA or said vitamins of the B-complex group do exert anti-depressant and anxiolytic activity in animal models.

Preferably, the composition for use according to the present invention has been formulated as an oral formulation such as a tablet, a chewing tablet, a capsule or a liquid, a suspension, or a sublingual formulation or any formulation for mucosal absorbance of the active ingredients of said composition, such as a lozenge or a quick dissolving powder, or a transdermal or topical formulation wherein the composition is administered in a cream, an ointment or a gel.

In a particular embodiment of the invention, the composition is provided as a tablet, wherein the tablet comprises PEA 300 mg, vitamin B1 1,1 mg, vitamin B2 1,4 mg, vitamin B3 16 mg, vitamin B5 6 mg, vitamin B6 1,5 mg, vitamin B8 50 mg.

The present invention will be illustrated further by means of the following examples.

### EXAMPLES

### Example 1

A female patient born in 1950, suffering from diabetes treated with metformin (N,N-Dimethylimidodicarbonimidic diamide) and insulin, presented with severe proximal asymmetrical neuropathic pains in left upper leg, pain score 10 on the Numeric Rating Scale (NRS). Patient was unable to walk and ride the bicycle. Treatment with amitriptyline induced drowsiness. The patient was treated with PEA 400 mg three times per day and the pain score decreased to 8. Vitamin B complex comprising vitamins B1, 2, 3, 5, 6, 8, folic acid and B12 at 300% of their recommended daily intake, was added. Pain scores decreased 2 more points and overall improvement in quality of life was 60%. She could again walk, ride the bicycle and take up social contacts.

### Example 2

Male patient, born in 1931 and suffering from diabetes type II, treated with metformin. Since 2 years he had increasing complaints of burning pain, itching and tingling of feet and legs. Therapy with PEA (1200 mg/day) was started for 1 month. This decreased the symptoms from a score of 8 to 7 on the NRS. Increasing PEA to 2400 mg/day improved scores to 5. Adding vitamin B complex comprising vitamins B1, 2, 3, 5, 6, 8, folic acid and B12, at a dose of 300% of the RDI / day, resulted in complete vanishing of all neuropathic positive symptoms: pain, tingling and itching.

### Example 3

Female patient born in 1953, suffering from chronic neuropathic pain due to a chronic idiopathic axonal neuropathy (CIAP), NRS score 8, could not be treated with pregabalin 75 mg twice daily, amitriptyline 20 mg before the night, nor duloxetine 600 mg, due to adverse reactions. The vitamin D level in the blood was below 50 nmol/liter and treatment was initiated with 2000 IE vitamin D/daily, 1200 mg PEA daily, together with a vitamin B complex, consisting of 300% of the RDI. Within 4 weeks the NRS decreased to 5 and after 4 more weeks the NRS was 3.

### Example 4

In this Example 4, we will describe a pain treatment program based on the PEA-vitamin B complex added to vitamin D (2000 IU) and omega 3 fatty acids (consisting of about 7 gram flaxseed oil/day).

In a group of 4 patients, suffering from chronic painful neuropathy, with pain scores of about 7-8, a treatment step by step program ('*stepped care program*') was initiated and completed in order to evaluate the synergistic aspects of the various components described herewith: PEA, vitamins B complex, vitamins D and vegetable oil).

Step 1 of the treatment regime consisted of 3 times daily 400 mg PEA. This treatment resulted in a decrease of pain in weeks 1-4 of about 25%.

Next step was to add the complete described group of vitamins B, also administered 3 times daily, to be taken together with PEA. The complete described group of vitamins B encompass vitamins B1 (Thiamine), B2 (Riboflavin), B3 (Niacin), B5 (Pantothenic acid), B6 (pyridoxine, pyridoxal, pyridoxamine), B7 (Biotin), B9 (Folic acid) and B12 (Cobalamin). This again resulted in further clinical relevant pain reduction.

Last step was to administer 2 drops of vitamin D3 (totally 2000 IU) in one tablespoon of flaxseed oil in the breakfast.

We recommended the breakfast defined by Budwig, as flaxseed oil is a part of this breakfast; this breakfast is dating back to the early 1950s, and was developed by Dr J. Budwig. Flaxseed oil is a vegetable oil rich in omega-3 fats, eicosapentaenoic acid (EPA) and docosahexaenoic acid (DHA). Flaxseed oil also contains a third, plant-based omega-3 oil, alpha-linolenic acid (ALA). In a separate program in stead of flaxseed oil, omega-3 fish oil capsules were prescribed, leading to comparable findings).

Pain reduction after adding vitamins D and flaxseed oil in the last step further improved and after 3 months, with this stepped care program a total pain reduction of 50% or more compared to baseline was achieved.

### Example 5

A male patient from 1961, 55 years old, since 3 months increasing pain in leg and back. An MRI scan showed a nervus ischiadicus compression syndrome L5/S1. His pain score increased steadily to about 8 on the NRS and then he started using PEA combined with the complete vitamin B complex, *i.e.* the group of vitamins B1 (Thiamine), B2 (Riboflavin), B3 (Niacin), B5 (Pantothenic acid), B6 (pyridoxine, pyridoxal, pyridoxamine), B7 (Biotin), B9 (Folic acid) and B12 (Cobalamin). After 3 days using 3 times daily 400 mg PEA and 3 times daily a total daily dose of the vitamin B complex of 150%, a clinical relevant decrease in pain of 50% was achieved. No side effects were observed or mentioned. Normally, based on clinical studies in nervus ischiadicus compression pain, pain scores decrease 30-50% but only after about 3 weeks. Surprisingly, in this patient 50% pain reduction was achieved in already 3 days.

### Example of PEA-vitamin B combinations

### Example a: excipient free combination in Vega caps

Excipient free capsules comprising PEA 400 mg, vitamin B1 1,1 mg, vitamin B2 1,4 mg, vitamin B3 16 mg, vitamin B5 6 mg, vitamin B6 1,5 mg, vitamin B8 50 microgram, folic acid 200 microgram, vitamin B12 2,5 microgram.

### Example b: PEA-B-complex matrix tablets

"PEA-B-complex matrix" tablets comprising PEA 300 mg, vitamin B1 1,1 mg, vitamin B2 1,4 mg, vitamin B3 16 mg, vitamin B5 6 mg, vitamin B6 1,5 mg, vitamin B8 50 microgram, folic acid 200 microgram, vitamin B12 2,5 microgram, cellulose 40 mg, lactose 120 mg, sodium carboxymethyl-amide 25 mg, precipitated silica 20 mg, polyvinylpyrrolidone 17 mg, maize starch 13 mg, Tween 20 7 mg, magnesium stearate 6 mg.

### Example c: PEA-B12-D3-borage oil gelatin capsules

"PEA-B12-D3-borage oil gelatin capsules" comprising PEA 300 mg, vitamin B12 2,5 microgram, 400 IE vitamin D3, lecithin 15 mg (E322), 50 mg borage seed oil, 5 mg Vitamin E.

### Example d: PEA-B-complex-D3-extra virgin olive oil capsules

"PEA- B-complex-D-extra virgin olive oil gelatin capsules" comprising PEA 300 mg, vitamin B1 1,1 mg, vitamin B2 1,4 mg, vitamin B3 16 mg, vitamin B5 6 mg, vitamin B6 1,5 mg, vitamin B8 50 microgram, folic acid 200 microgram, vitamin B12 2,5 microgram, 400 IE vitamin D3, 200 mg extra virgin olive oil, 5 mg Vitamin E. Example e: PEA-B12-extra virgin olive oil capsules

"PEA-extra virgin olive oil capsules" comprising of PEA 300 mg, vitamin B12 2,5 microgram and 500 mg extra virgin olive oil.

### Example f: cream

Cream comprising PEA 1,5% by weight in a base of buffered cetomacrogol cream, together with 1% B12 by weight and 5% amitriptyline by weight.

### Example g: PEA in olive oil

A fat in (olive)-oil matrix of palmitoylethanolamide was prepared according to an embodiment of the invention. See Figure 1, a microscopic photograph of the resulting fat in (olive)-oil matrix of palmitoylethanolamide (10%: palmitoylethanolamide dissolves in hot oil resulting in a semi-solid fatty-oil matrix).

## Claims

1. A composition comprising palmitoylethanolamide and one or more vitamins of the vitamin B complex or salts thereof, wherein the composition comprises between 30% and 3000% of the recommended daily intake of vitamin B, preferably between 50% and 1000%, per unit of composition.

2. Composition according to claim 1, wherein palmitoylethanolamide and the one or more vitamins of the vitamin B complex or salts thereof are the only pharmaceutically active ingredients in the composition.

3. Composition according to claim 1, wherein the one or more vitamins of the vitamin B complex is, or are, respectively, chosen from the group consisting of vitamin B1, vitamin B2, vitamin B3, vitamin B5, vitamin B8, folic acid, vitamin B12 or mixtures thereof.

4. Composition according to claim 2, wherein the one or more vitamins of the vitamin B complex is, or are, respectively, chosen from the group consisting of vitamin B1, vitamin B2, vitamin B3, vitamin B5, vitamin B8, folic acid, vitamin B12 or mixtures thereof.

5. Composition according to claim 1, wherein the one or more vitamins of the vitamin B complex is, or are, respectively, chosen from the group consisting of vitamin B1, vitamin B2, vitamin B3, vitamin B5, vitamin B6, vitamin B8, folic acid, vitamin B12 or mixtures thereof.

6. Composition according to claim 2, wherein the one or more vitamins of the vitamin B complex is, or are, respectively, chosen from the group consisting of vitamin B1, vitamin B2, vitamin B3, vitamin B5, vitamin B6, vitamin B8, folic acid, vitamin B12 or mixtures thereof.

7. Composition according to any of the claims 1, 3 or 5, wherein the composition comprises between 100 and 800 mg palmitoylethanolamide, preferably between 300 mg and 600 mg palmitoylethanolamide, per unit of composition; optionally, containing said palmitoylethanolamide in an oil matrix, in a volume ratio of 5%-20% (volume/volume), preferably 10% (volume/volume) palmitoylethanolamide / olive oil.

8. Composition according to any of the claims claim 2, 4 or 6, wherein the composition comprises between 100 and 800 mg palmitoylethanolamide, preferably between 300 mg and 600 mg palmitoylethanolamide, per unit of composition; optionally, containing said palmitoylethanolamide in an oil matrix, in a volume ratio of 5%-20% (volume/volume), preferably 10% (volume/volume) palmitoylethanolamide / olive oil.

9. Composition according to any of the claims 1, 3, 5 or 7, wherein the composition further comprises a vegetable oil and/or vitamin D.

10. Composition according to claim 7, wherein the composition comprises between 50 and 150 mg vegetable oil per unit of composition.

11. Composition according to claim 9 or 10, wherein the composition comprises between 10 and 40 microgram vitamin D3 per unit of composition.

12. Composition according to any of the claims 1 to 11, wherein the composition is formulated as a pharmaceutical composition or a nutraceutical composition.

13. Composition according to any of the previous claims, wherein the composition is formulated as an oral formulation, a sublingual formulation, a transdermal or a topical formulation.

14. A composition according to claim 13, wherein the formulation is a single compounded cream or gel comprising a cream basis or gel basis selected from Cremor cetomacrogolis FNA or Lanette cream FNA.

15. A composition according to any of the claims 1, 3, 5, 7 or 9-11, wherein the composition further comprises one or more analgesic compounds, preferably the composition comprises 1-20% by weight of one or more analgesic compounds.

16. A composition according to claim 15, wherein the composition comprises ketamine, gabapentin, pregabalin, amitriptyline, duloxetine, baclofen or a combination of any of the these analgesic compounds.

17. A method for preparing the composition according to any of the claims 1 to 16.

18. A method according to claim 17, wherein the palmitoylethanolamide and one or more vitamins of the vitamin B complex or salts thereof have been granulated into particles with an average particle size (D50) of between 10 and 1000 micron, preferably between 10 and 100 micron.

19. A method according to claim 17 or 18, comprising the step of adding the palmitoylethanolamide particles to an oil, preferably any vegetable oil or any fish oil comprising omega-3 fatty acids, preferably olive oil, such that a composition comprising palmitoylethanolamide in an oil matrix, in a volume ratio of 5%-20% (volume/volume), preferably 10% (volume/volume) palmitoylethanolamide / vegetable oil, preferably palmitoylethanolamide / olive oil, is obtained.

20. A composition according to any of the claims 1 to 16, for use as a medicament.

21. A composition according to any of the claims 1-16 for use in pain alleviation.

22. A composition for use according to claim 21, wherein the pain is neuropathic pain.

23. A composition for use according to claim 21 or 22, wherein the neuropathic pain coincides with an axonal neuropathy such as diabetic neuropathy, a demyelinating neuropathy, chemotherapy induced neuropathy, HIV neuropathy, post herpetic neuropathy or postoperative pain.

24. A composition for use according to any of the claims 20 to 23, wherein palmitoylethanolamide and the one or more vitamins of the vitamin B complex or salts thereof are the only pharmaceutically active ingredients in the composition.

25. A composition for use according to any of the claims 21 to 23, wherein the composition further comprises an analgesic drug selected from the classes of antidepressants, anti-epileptics or opioids.

26. A composition for use according to claim 25, wherein the analgesic drug is selected from amitriptyline, duloxetine, pregabalin, gabapentin, tramadol, oxycontin and tapentadol.

## Patentansprüche

1. Zusammensetzung, welche Palmitoylethanolamid und ein oder mehrere Vitamine des Vitamin B-Komplexes oder Salze davon aufweist, wobei die Zusammensetzung zwischen 30 % und 3000 % der empfohlenen täglichen Einnahmemenge von Vitamin B aufweist, vorzugsweise zwischen 50 % und 1000 % pro Einheit der Zusammensetzung.

2. Zusammensetzung nach Anspruch 1, wobei Palmitoylethanolamid und das eine oder die mehreren Vitamine des Vitamin B-Komplexes oder Salze davon die einzigen pharmazeutisch aktiven Bestandteile in der Zusammensetzung sind.

3. Zusammensetzung nach Anspruch 1, wobei das eine oder die mehreren Vitamine des Vitamin B-Komplexes aus der Gruppe ausgewählt ist oder sind, die aus Vitamin B1, Vitamin B2, Vitamin B3, Vitamin B5, Vitamin B8, Folsäure, Vitamin B12 oder Mischungen davon besteht.

4. Zusammensetzung nach Anspruch 2, wobei das eine oder die mehreren Vitamine des Vitamin B-Komplexes aus der Gruppe ausgewählt ist oder sind, die aus Vitamin B1, Vitamin B2, Vitamin B3, Vitamin B5, Vitamin B8, Folsäure, Vitamin B12 oder Mischungen davon besteht.

5. Zusammensetzung nach Anspruch 1, wobei das eine oder die mehreren Vitamine des Vitamin B-Komplexes aus der Gruppe ausgewählt ist oder sind, die aus Vitamin B1, Vitamin B2, Vitamin B3, Vitamin B5, Vitamin B6, Vitamin B8, Folsäure, Vitamin B12 oder Mischungen davon besteht.

6. Zusammensetzung nach Anspruch 2, wobei das eine oder die mehreren Vitamine des Vitamin B-Komplexes aus der Gruppe ausgewählt ist oder sind, die aus Vitamin B1, Vitamin B2, Vitamin B3, Vitamin B5, Vitamin B6, Vitamin B8, Folsäure, Vitamin B12 oder Mischungen davon besteht.

7. Zusammensetzung nach einem der Ansprüche 1, 3 oder 5, wobei die Zusammensetzung zwischen 100 und 800 mg Palmitoylethanolamid, vorzugsweise zwischen 300 mg und 600 mg Palmitoylethanolamid, und zwarpro Einheit der Zusammensetzung, aufweist; wobei sie optional das Palmitoylethanolamid in einer Ölmatrix enthält, und zwar in einem Volumenverhältnis von 5% bis 20% (Volumen / Volumen), vorzugsweise 10% (Volumen / Volumen) Palmitoylethanolamid / Olivenöl aufweist.

8. Zusammensetzung nach einem der Ansprüche 2. 4 oder 6, wobei die Zusammensetzung zwischen 100 und 800 mg Palmitoylethanolamid, vorzugsweise zwischen 300 mg und 600 mg Palmitoylethanolamid, pro Einheit der Zusammensetzung aufweist; wobei sie optional das Palmitoylethanolamid in einer Ölmatrix in einem Volumenverhältnis von 5% bis 20% (Volumen / Volumen), vorzugsweise 10% (Volumen / Volumen) Palmitoylethanolamid / Olivenöl aufweist.

9. Zusammensetzung nach einem der Ansprüche 1, 3, 5 oder 7, wobei die Zusammensetzung ferner ein Pflanzenöl und / oder Vitamin D aufweist.

10. Zusammensetzung nach Anspruch 7, wobei die Zusammensetzung zwischen 50 und 150 mg Pflanzenöl pro Einheit der Zusammensetzung aufweist.

11. Zusammensetzung nach Anspruch 9 oder 10, wobei die Zusammensetzung zwischen 10 und 40 Mikrogramm Vitamin D3 pro Einheit der Zusammensetzung aufweist.

12. Zusammensetzung nach einem der Ansprüche 1 bis 11, wobei die Zusammensetzung als eine pharmazeutische Zusammensetzung oder als eine nutrazeutische Zusammensetzung formuliert ist.

13. Zusammensetzung nach einem der vorhergehenden Ansprüche, wobei die Zusammensetzung als eine orale Formulierung bzw. ein orales Präparat, ein sublinguales Präparat, ein transdermales oder ein topisches Präparat formuliert ist.

14. Zusammensetzung nach Anspruch 13, wobei die Formulierung bzw. das Präparat eine einzeln zusammengesetzte Creme oder ein Gel ist, die eine Cremebasis oder Gelbasis aufweist, die aus Cremor Cetomacrogolis-FNA oder Lanette-Creme-FNA ausgewählt ist.

15. Zusammensetzung nach einem der Ansprüche 1, 3, 5, 7 oder 9-11, wobei die Zusammensetzung ferner eine oder mehrere analgetische Verbindungen aufweist, wobei die Zusammensetzung vorzugsweise 1 - 20 Gewichts-% von einer oder mehreren analgetischen Verbindungen aufweist.

16. Zusammensetzung nach Anspruch 15, wobei die Zusammensetzung Ketamin, Gabapentin, Pregabalin, Amitriptylin, Duloxetin, Baclofen oder eine Kombination irgendwelcher dieser analgetischen Verbindungen aufweist.

17. Verfahren zur Herstellung der Zusammensetzung nach einem der Ansprüche 1 bis 16.

18. Verfahren nach Anspruch 17, wobei das Palmitoylethanolamid und ein oder mehrere Vitamine des Vitamin B-Komplexes oder Salze davon zu Partikeln mit einer durchschnittlichen Partikelgröße (D50) zwischen 10 und 1000 Mikrometern, vorzugsweise zwischen 10 und 100 Mikrometern, granuliert wurden.

19. Verfahren nach Anspruch 17 oder 18, welches den Schritt des Zugebens der Palmitoylethanolamid-Partikel zu einem Öl aufweist, vorzugsweise irgendeinem Pflanzenöl oder irgendeinem Fischöl, welches Omega-3-Fettsäuren aufweist, vorzugsweise Olivenöl, so dass eine Zusammensetzung, die Palmitoylethanolamid in einer Ölmatrix aufweist, in einem Volumenverhältnis von 5% bis 20% (Volumen / Volumen), vorzugsweise 10% (Volumen / Volumen) Palmitoylethanolamid / Pflanzenöl, vorzugsweise Palmitoylethanolamid / Olivenöl, erhalten wird.

20. Zusammensetzung nach einem der Ansprüche 1 bis 16 zur Verwendung als ein Medikament.

21. Zusammensetzung nach einem der Ansprüche 1 bis 16 zur Verwendung zur Schmerzlinderung.

22. Zusammensetzung zur Verwendung nach Anspruch 21, wobei der Schmerz ein neuropathischer Schmerz ist.

23. Zusammensetzung zur Verwendung nach Anspruch 21 oder 22, wobei der neuropathische Schmerz mit einer axonalen Neuropathie zusammenhängt, wie diabetische Neuropathie, eine demyelinisierende Neuropathie, eine durch Chemotherapie induzierte Neuropathie, eine HIV-Neuropathie, eine postherpetische Neuropathie oder postoperativer Schmerz.

24. Zusammensetzung zur Verwendung nach einem der Ansprüche 20 bis 23, wobei Palmitoylethanolamid und das eine oder die mehreren Vitamine des Vitamin B-Komplexes oder Salze davon die einzigen pharmazeutisch aktiven Bestandteile in der Zusammensetzung sind.

25. Zusammensetzung zur Verwendung nach einem der Ansprüche 21 bis 23, wobei die Zusammensetzung ferner ein analgetisches Arzneimittel aufweist, das aus den Klassen der Antidepressiva, Antiepileptika oder Opioiden ausgewählt ist.

26. Zusammensetzung zur Verwendung nach Anspruch 25, wobei das analgetische Arzneimittel aus Amitriptylin, Duloxetin, Pregabalin, Gabapentin, Tramadol, Oxycontin und Tapentadol ausgewählt ist.

## Revendications

1. Composition comprenant du palmitoyléthanolamide et une ou plusieurs vitamines du complexe de vitamine B ou de sels de celui-ci, dans laquelle la composition comprend de 30 % à 3000 % de la dose quotidienne recommandée de vitamine B, de préférence de 50 % à 1000 %, par unité de composition.

2. Composition selon la revendication 1, dans laquelle le palmitoyléthanolamide et les une ou plusieurs vitamines du complexe de vitamine B ou des sels de celui-ci sont les seuls ingrédients pharmaceutiquement actifs de la composition.

3. Composition selon la revendication 1, dans laquelle les une ou plusieurs vitamines du complexe de vitamine B est ou sont respectivement choisies dans le groupe constitué par la vitamine B1, la vitamine B2, la vitamine B3, la vitamine B5, la vitamine B8, l'acide folique, la vitamine B12 ou des mélanges de ceux-ci.

4. Composition selon la revendication 2, dans laquelle les une ou plusieurs vitamines du complexe de vitamine B est ou sont respectivement choisies dans le groupe constitué par la vitamine B1, la vitamine B2, la vitamine B3, la vitamine B5, la vitamine B8, l'acide folique, la vitamine B12 ou des mélanges de ceux-ci.

5. Composition selon la revendication 1, dans laquelle les une ou plusieurs vitamines du complexe de vitamine B est ou sont respectivement choisies dans le groupe constitué par la vitamine B1, la vitamine B2, la vitamine B3, la vitamine B5, la vitamine B6, la vitamine B8, l'acide folique, la vitamine B12 ou des mélanges de ceux-ci.

6. Composition selon la revendication 2, dans laquelle les une ou plusieurs vitamines du complexe de vitamine B est ou sont respectivement choisies dans le groupe constitué par la vitamine B1, la vitamine B2, la vitamine B3, la vitamine B5, la vitamine B6, la vitamine B8, l'acide folique, la vitamine B12 ou des mélanges de ceux-ci.

7. Composition selon l'une quelconque des revendications 1, 3 ou 5, dans laquelle la composition comprend entre 100 et 800 mg de palmitoyléthanolamide, de préférence entre 300 mg et 600 mg de palmitoyléthanolamide, par unité de composition ;
contenant facultativement ledit palmitoyléthanolamide dans une matrice huileuse, en un rapport volumique de 5 % à 20 % (volume/volume), de préférence 10 % (volume/volume) de palmitoyléthanolamide/huile d'olive.

8. Composition selon l'une quelconque des revendications 2, 4 ou 6, dans laquelle la composition comprend entre 100 et 800 mg de palmitoyléthanolamide, de préférence entre 300 mg et 600 mg de palmitoyléthanolamide, par unité de composition ;
contenant facultativement ledit palmitoyléthanolamide dans une matrice huileuse, en un rapport volumique de 5 % à 20 % (volume/volume), de préférence 10 % (volume/volume) de palmitoyléthanolamide/huile d'olive.

9. Composition selon l'une quelconque des revendications 1, 3, 5 ou 7, dans laquelle la composition comprend en outre une huile végétale et/ou de la vitamine D.

10. Composition selon la revendication 7, dans laquelle la composition comprend entre 50 et 150 mg d'huile végétale par unité de composition.

11. Composition selon la revendication 9 ou 10, dans laquelle la composition comprend entre 10 et 40 microgrammes de vitamine D3 par unité de composition.

12. Composition selon l'une quelconque des revendications 1 à 11, dans laquelle la composition est formulée en tant que composition pharmaceutique ou composition nutraceutique.

13. Composition selon l'une quelconque des revendications précédentes, dans laquelle la composition est formulée sous la forme d'une formulation orale, d'une formulation sublinguale, d'une formulation transdermique ou topique.

14. Composition selon la revendication 13, dans laquelle la formulation est une crème ou un gel composé(e) unique comprenant une base de crème ou une base de gel sélectionnée parmi Cremor Cetomacrogolis FNA ou la crème de Lanette FNA.

15. Composition selon l'une quelconque des revendications 1, 3, 5, 7 ou 9 à 11, dans laquelle la composition comprend en outre un ou plusieurs composés analgésiques, de préférence la composition comprend 1 à 20 % en poids d'un ou plusieurs composés analgésiques.

16. Composition selon la revendication 15, dans laquelle la composition comprend de la kétamine, de la gabapentine, de la prégabaline, de l'amitriptyline, de la duloxétine, du baclofène ou une combinaison de l'un quelconque de ces composés analgésiques.

17. Procédé de préparation de la composition selon l'une quelconque des revendications 1 à 16.

18. Procédé selon la revendication 17, dans lequel le palmitoyléthanolamide et une ou plusieurs vitamines du complexe de la vitamine B ou de sels de celui-ci ont été granulés en particules ayant une taille de particule moyenne (D50) comprise entre 10 et 1000 microns, de préférence entre 10 et 100 microns.

19. Procédé selon la revendication 17 ou 18, comprenant l'étape consistant à ajouter les particules de palmitoyléthanolamide à une huile, de préférence toute huile végétale ou toute huile de poisson comprenant des acides gras oméga-3, de préférence de l'huile d'olive, de telle sorte qu'une composition comprenant du palmitoyléthanolamide dans une matrice d'huile, en un rapport volumique de 5 % à 20 % (volume/volume), de préférence de 10 % (volume/volume) de palmitoyléthanolamide/huile végétale, de préférence de palmitoyléthanolamide/huile d'olive est obtenue.

20. Composition selon l'une quelconque des revendications 1 à 16, destinée à être utilisée comme médicament.

21. Composition selon l'une quelconque des revendications 1 à 16, destinée à être utilisée pour soulager la douleur.

22. Composition à utiliser selon la revendication 21, dans laquelle la douleur est une douleur neuropathique.

23. Composition à utiliser selon la revendication 21 ou 22, dans laquelle la douleur neuropathique coïncide avec une neuropathie axonale telle qu'une neuropathie diabétique, une neuropathie démyélinisante, une neuropathie induite par une chimiothérapie, une neuropathie du VIH, une neuropathie post-herpétique ou une douleur postopératoire.

24. Composition à utiliser selon l'une quelconque des revendications 20 à 23, dans laquelle le palmitoyléthanolamide et les une ou plusieurs vitamines du complexe de vitamine B ou de sels de celui-ci sont les seuls ingrédients pharmaceutiquement actifs de la composition.

25. Composition à utiliser selon l'une quelconque des revendications 21 à 23, dans laquelle la composition comprend en outre un médicament analgésique choisi parmi les classes d'antidépresseurs, d'antiépileptiques ou d'opioïdes.

26. Composition à utiliser selon la revendication 25, dans laquelle le médicament analgésique est choisi parmi l'amitriptyline, la duloxétine, la prégabaline, la gabapentine, le tramadol, l'oxycontin et le tapentadol.
